# EUROPEAN PATENT SPECIFICATION

(11) **EP 1 846 347 B1**
(45) Date of publication and mention of the grant of the patent: **30.05.2012**
(21) Application number: 06710303.6
(22) Date of filing: 01.02.2006
(51) Int. Cl.: C07B 59/00, C07C 233/11, C07C 57/30, C07C 69/612, C07D 295/182

(54) **METHOD FOR THE USE OF [11C] CARBON MONOXIDE IN LABELING SYNTHESIS OF 11C-LABELLED COMPOUNDS BY THERMALLY INDUCED FREE RADICAL CARBONYLATION**
VERFAHREN ZUR VERWENDUNG VON [11C]KOHLENMONOXYD ZUR MARKIERENDEN SYNTHESE VON 11C-MARKIERTEN VERBINDUNGEN DURCH THERMISCH INDUZIERTE RADIKALISCHE CARBONILIERUNG
PROCEDE D'UTILISATION DE MONOXYDE DE CARBONE [11C] DANS LA SYNTHESE DE MARQUAGE DE COMPOSE MARQUES AU 11C PAR CARBONYLATION RADICALAIRE INDUITE THERMIQUEMENT

(30) Priority: 01.02.2005 US 648830 P
(43) Date of publication of application: 24.10.2007
(73) Proprietor: GE Healthcare Limited, Little Chalfont Buckinghamshire HP7 9NA (GB)
(72) Inventor: LANGSTROM, Bengt, Uppsala Research Imaging, S-751-85 Uppsala (SE); ITSENKO, Oleksiy, Department of Organic Chemistry, S-751 24 Uppsala (SE); KIHLBERG, Tor, Uppsala Research Imaging, S-751 85 Uppsala (SE)
(74) Representative: Canning, Lewis R.
(86) International application number: PCT/IB2006/000185
(87) International publication number: WO 2006/082497

(56) References cited:
- EP-A- 1 444 990
- WO-A-02/102711
- WO-A-03/010175
- WO-A-2005/042441
- WO-A-2006/051428
- ITSENKO, OLEKSIY ET AL: "Photoinitiated Carbonylation with [11C]Carbon Monoxide Using Amines and Alkyl Iodides" JOURNAL OF ORGANIC CHEMISTRY, vol. 69, no. 13, 2004, pages 4356-4360, XP002385440
- RYU I ET AL: "Metal catalyst-free by design. The synthesis of amides from alkyl iodides carbon monoxide and amines by a hybrid radical/ionic reaction" CHEMICAL COMMUNICATIONS - CHEMCOM, ROYAL SOCIETY OF CHEMISTRY, GB, 1998, pages 1953-1954, XP002319932 ISSN: 1359-7345
- I. RYU: "Radical carbonylation of iodoalkanes and saturated alcohols using carbon monoxide" CHEM. SOC. REV.,, vol. 30, 2001, pages 16-25, XP009067866
- I. RYU, N. SONODA: "Free-Radical Carbonylation: Then and now" ANGEW. CHEM. INT. ED. ENGL., vol. 35, 1996, pages 1051-1066, XP002385442
- KIYOTO NAGAHARA ET AL.: "Radical Carboxylation: Ester Synthesis from Alkyl Iodides, Carbon Monoxides, and Alcohols under Irradaiation Conditions" J. AM CHEM SOC., vol. 119, no. 23, 1997, pages 5465-5466, XP002385441
- RAHMAN O ET AL: "Aryl Triflates and 11C/13C Carbon Monoxide in the Synthesis of 11C/13C Amides" JOURNAL OF ORGANIC CHEMISTRY, AMERICAN CHEMICAL SOCIETY. EASTON, US, vol. 68, 9 May 2003 (2003-05-09), pages 3558-3562, XP002319931 ISSN: 0022-3263
- KIHLBERG T ET AL: "Biologically Active C-Labeled Amides Using Palladium-Mediated Reactions with Aryl Halides and [11C]Carbon Monoxide" JOURNAL OF ORGANIC CHEMISTRY, AMERICAN CHEMICAL SOCIETY. EASTON, US, vol. 64, 1999, pages 9201-9205, XP002168717 ISSN: 0022-3263 cited in the application
- F. KARIMI, B. LANGSTRÖM: "Synthesis of 11C-Labelled Amides by Palladium-Mediated Carboxamination Using [11C]Carbon monoxide, in situ Activated Amines and 1,2,2,6,6-Pentapiperidine" EURPOEAN JOURNAL OF ORGANIC CHEMISTRY, 2003, pages 2132-2137, XP002383784
- F. KARIMI, B. LANGSTRÖM: "Synthesis of 11C-amides using [11C]-carbon monoxide and in situ activated amines by palladium-mediated carboxaminations" ORG. BIOMOL. CHEM., vol. 1, 2003, pages 541-546, XP002383785
- DATABASE BEILSTEIN BEILSTEIN INSTITUTE FOR ORGANIC CHEMISTRY, FRANKFURT-MAIN, DE; XP002385454 Database accession no. BRN 1699477 & DAVENPORT ET AL.: J. LABELLED COMPD. RADIOPHARM., vol. 40, 1997, pages 309-311,
- DATABASE BEILSTEIN BEILSTEIN INSTITUTE FOR ORGANIC CHEMISTRY, FRANKFURT-MAIN, DE; XP002385455 Database accession no. BRN 8829315 & KIHLBERG, T.; LAENGSTROEM, B.: J. LABELLED COMPD. RADIOPHARM., vol. 44, 2001, pages S990-S992,
- DATABASE BEILSTEIN BEILSTEIN INSTITUTE FOR ORGANIC CHEMISTRY, FRANKFURT-MAIN, DE; XP002385456 Database accession no. BRN 2104069 & ELLIOT, HANAM: BIOCHEM. J., 1968, page 551, 552,
- DATABASE BEILSTEIN BEILSTEIN INSTITUTE FOR ORGANIC CHEMISTRY, FRANKFURT-MAIN, DE; XP002385457 Database accession no. BRN 8311991 & PERRIO-HUARD, C. ET AL.: J. LABELLED COMPD. RADIOPHARM., vol. 42, no. 1, 1999, pages S92-S93,
- DATABASE BEILSTEIN BEILSTEIN INSTITUTE FOR ORGANIC CHEMISTRY, FRANKFURT-MAIN, DE; XP002385458 Database accession no. BRN 1701671 & BUCHANAN ET AL.: J. BIOL. CHEM., 150, 1943, page 414,
- DATABASE BEILSTEIN BEILSTEIN INSTITUTE FOR ORGANIC CHEMISTRY, FRANKFURT-MAIN, DE; XP002385459 Database accession no. BRN 1938559 & AZIM, ELMOSTAFA ET AL.: J. LABELLED COMPD. RADIOPHARM., vol. 39, no. 11, 1997, pages 907-914,
- ITSENKO, OLEKSIY ET AL: "Radical -mediated carboxylation of alkyl iodides with [11C]carbon monoxide in solvent mixtures" JOURNAL OF ORGANIC CHEMISTRY, vol. 70, no. 6, 2005, pages 2244-2249, XP002385443
- ITSENKO, OLEKSIY ET AL: "Photoinitiated Free Radical Carbonylation Enhanced by Photosensitizers" ORGANIC LETTERS, vol. 7, no. 21, 2005, pages 4661-4664, XP002385444
- ITSENKO, OLEKSIY ET AL: "Carboxylation of alkyl iodides with [11C] and (13C)carbon monoxide. Using sulfoxides as oxygen nucleophiles" SYNLETT, no. 20, 2005, pages 3154-3156, XP002385445
- ITSENKO, OLEKSIY ET AL: "Synthesis of aliphatic [carbonyl-11C] esters using [11C]carbon monoxide" EUROPEAN JOURNAL OF ORGANIC CHEMISTRY, no. 17, 2005, pages 3830-3834, XP002385446
- Michael J. Welch and Carol S. Redvanly: "Handbook of Radiopharmaceuticals", 2003, John Wiley and Sons, England

## Description

### Field of the Invention

The present invention relates to a method for the use of carbon-isotope monoxide in labeling synthesis. More specifically, the invention relates to a method for producing an [¹¹C]carbon monoxide enriched gas mixture from an initial [¹¹C]carbon dioxide gas mixture, and using the produced gas mixture in labeling synthesis by thermally initiated carbonylation. Radiolabeled compounds, for example, amides, acids, or esters, are provided using alkyl or aryl iodides and appropriate nucleophiles, for example, amines, water or alcohols, as precursors. The radiolabeled compounds according to the present invention are useful as radiopharmaceuticals, specifically for use in Positron Emission Tomography (PET).

### Background of the Invention

Tracers labeled with short-lived positron emitting radionuclides (e.g. ¹¹C, t_{1/2} = 20.3 min) are frequently used in various non-invasive in vivo studies in combination with positron emission tomography (PET). Because of the radioactivity, the short half-lives and the submicromolar amounts of the labeled substances, extraordinary synthetic procedures are required for the production of these tracers. An important part of the elaboration of these procedures is development and handling of new ¹¹C-labeled precursors. This is important not only for labeling new types of compounds, but also for increasing the possibility of labeling a given compound in different positions.

During the last two decades carbonylation chemistry using carbon monoxide has developed significantly. The recent development of methods such as palladium-catalyzed carbonylative coupling reactions has provided a mild and efficient tool for the transformation of carbon monoxide into different carbonyl compounds.

Carbonylation reactions using [¹¹C]carbon monoxide has a primary value for PET-tracer synthesis since biologically active substances often contain carbonyl groups or functionalities that can be derived from a carbonyl group. The syntheses are tolerant to most functional groups, which means that complex building blocks can be assembled in the carbonylation step to yield the target compound. This is particularly valuable in PET-tracer synthesis where the unlabeled substrates should be combined with the labeled precursor as late as possible in the reaction sequence, in order to decrease synthesis-time and thus optimize the uncorrected radiochemical yield.

When compounds are labeled with ¹¹C, it is usually important to maximize specific radioactivity. In order to achieve this, the isotopic dilution and the synthesis time must be minimized. Isotopic dilution from atmospheric carbon dioxide may be substantial when [¹¹C]carbon dioxide is used in a labeling reaction. Due to the low reactivity and atmospheric concentration of carbon monoxide (0.1 ppm vs. 3.4 x 10⁴ ppm for CO₂), this problem is reduced with reactions using [¹¹C]carbon monoxide.

The synthesis of [¹¹C]carbon monoxide from [¹¹C]carbon dioxide using a heated column containing reducing agents such as zinc, charcoal or molybdenum has been described previously in several publications. Although [¹¹C]carbon monoxide was one of the first ¹¹C-labelled compounds to be applied in tracer experiments in human, it has until recently not found any practical use in the production of PET-tracers. One reason for this is the low solubility and relative slow reaction rate of [¹¹C]carbon monoxide which causes low trapping efficiency in reaction media. The general procedure using precursors such as [¹¹C]methyl iodide, [¹¹C]hydrogen cyanide or [¹¹C]carbon dioxide is to transfer the radioactivity in a gas-phase, and trap the radioactivity by leading the gas stream through a reaction medium. Until recently this has been the only accessible procedure to handle [¹¹C]carbon monoxide in labeling synthesis. With this approach, the main part of the labeling syntheses with [¹¹C]carbon monoxide can be expected to give a very low yield or fail completely.

There are only a few examples of practically valuable ¹¹C-labelling syntheses using high pressure techniques (> 300 bar). In principal, high pressures can be utilized for increasing reaction rates and minimizing the amounts of reagents. One problem with this approach is how to confine the labeled precursor in a small high-pressure reactor. Another problem is the construction of the reactor. If a common column type of reactor is used (i.e. a cylinder with tubing attached to each end), the gas-phase will actually become efficiently excluded from the liquid phase at pressurization. The reason is that the gas-phase, in contracted form, will escape into the attached tubing and away from the bulk amount of the liquid reagent.

The cold-trap technique is widely used in the handling of ¹¹C-labelled precursors, particularly in the case of [¹¹C]carbon dioxide. The procedure has, however, only been performed in one single step and the labeled compound was always released in a continuous gas-stream simultaneous with the heating of the cold-trap. Furthermore, the volume of the material used to trap the labeled compound has been relative large in relation to the system to which the labeled compound has been transferred. Thus, the option of using this technique for radical concentration of the labeled compound and miniaturization of synthesis systems has not been explored. This is especially noteworthy in view of the fact that the amount of a ¹¹C-labelled compound usually is in the range 20-60 nmol.

Recent technical development for the production and use of [¹¹C] carbon monoxide has made this compound useful in labeling synthesis. WO 02/102711 describes a system and a method for the production and use of a carbon-isotope monoxide enriched gas-mixture from an initial carbon-isotope dioxide gas mixture. [¹¹C] carbon monoxide may be obtained in high radiochemical yield from cyclotron produced [¹¹C] carbon dioxide and can be used to yield target compounds with high specific radioactivity. This reactor overcomes the difficulties listed above and is useful in synthesis of ¹¹C-labelled compounds using [¹¹C] carbon monoxide in palladium or selenium mediated reaction. With such method, a broad array of carbonyl compounds can be labeled (Kilhlberg, T.; Langstrom, B. J., Org. Chem. 64, 1999, 9201-9205, Kihlberg, T., Karimi, F., Langstrom, B., J. Org. Chem. 67, 2002, 3687-3692).

Transition-metal mediated chemistry, however, do not allow to use alkyl halides (with rare exceptions) as precursors for labeling due to facile β-elimination. To overcome this limitation, a method based on the photo-induced radical-mediated carbonylation with [¹¹C]carbon monoxide has been developed and a special reaction chamber has been constructed (Itsenko et al J Org Chem 2004; 69: 4356-4360). This method further increased the utility of [¹¹C]carbon monoxide as a labeling agent for providing biologically active ¹¹C-labelled compounds for PET studies.

This method, however, does not work well in some cases, as precursors or labeled products may be photosensitive compounds, which may be destroyed with the photo-irradiation, which is used for initiating of the chemical transformation.

On the other hand, the use of alternative, thermal induction of the radical reactions may be the way to avoid these problems, and, moreover, provides a possibility to run labeling syntheses using a simpler, window-free, reactor, the same type as the one that has been used for palladium-mediated labeling reactions. Thermal induction of the radical reaction has been applied for non-radioactive reactions (Ryu et al Chem Commun 1998; 1953-1954 and Ryu 2001 Chem Soc Rev 2001; 30: 16-25)

Therefore, there is a need for a method in order to perform thermally induced free radical carbonylation reactions with [¹¹C] carbon monoxide to circumvent the problems that may arise from UV irradiation of the labeling precursors and labeled products, thus extending the scope of applicability of the radical-mediated reactions and provide target structures with high yield to further increase the utility of [¹¹C] carbon monoxide in preparing useful PET tracers.

### Summary of the Invention

The present invention provides a method for labeling synthesis, comprising:
a) providing a high pressure reaction chamber having a liquid inlet and a gas inlet in a bottom surface thereof,
b) providing a reagent volume to be labeled comprising a thermal radical initiator A, mediator M, alkyl or aryl iodide RI and a nucleophile NuH,
c) introducing a carbon-isotope monoxide enriched gas-mixture into the reaction chamber via the gas inlet,
d) introducing, at high pressure, said reagent volume into the reaction chamber via the liquid inlet,
e) heating the reaction chamber and waiting a predetermined time while the labeling synthesis occurs,
f) collecting a solution of labeled compound of Formula (III) from the reaction chamber: 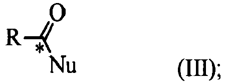 wherein:
R is linear or cyclic alkyl or substituted alkyl, aryl or substituted aryl and may contain chloro-, fluoro-, keto-, and carboxyl groups, which are separated by at least one carbon atom from the carbon atom bearing the iodide atom, and Nu is a deprotonated nucleophile;
wherein high pressure is defined as >30 MPa (>300 bar); and,
wherein the carbon-isotope monoxide is [¹¹C]carbon monoxide.

The present invention provides a method for the synthesis of labeled compounds, for example, amides, acids, or esters, using thermally initiated carbonylation with [¹¹C] carbon monoxide using amines, water, or alcohols, respectively, and alkyl or aryl iodides.

### Brief Description of the Figures

Fig. 1 shows a flow chart over the method according to the invention.
Fig. 2 is a schematic view of a carbon-isotope monoxide production and labeling-system.
Fig. 3a and 3b show alternative embodiments of a reaction chamber used in the invention.

### Detailed Description of the Invention

One object of the invention is to provide a method for production of of carbon-isotope monoxide in labeling synthesis that overcomes the drawbacks of the prior art devices. This is achieved by the method described in the invention.

One advantage with such a method is that nearly quantitative conversion of carbon-isotope monoxide into labeled products can be accomplished.

There are several other advantages with the present method. The high-pressure technique makes it possible to use low boiling solvents such as diethyl ether at high temperatures (e.g. 200 °C). The use of a closed system consisting of materials that prevents gas diffusion, increases the stability of sensitive compounds and could be advantageous also with respect to Good Manufacturing Practice (GMP).

Still other advantages are achieved in that the resulting labeled compound is highly concentrated, and that the miniaturization of the synthesis system facilitates automation, rapid synthesis and purification, and optimization of specific radioactivity through minimization of isotopic dilution.

Most important is the opening of completely new synthesis possibilities, as exemplified by the present invention.

Embodiments of the invention will now be described with reference to the figures.

The term carbon-isotope that is used throughout this application refers to ¹¹C.

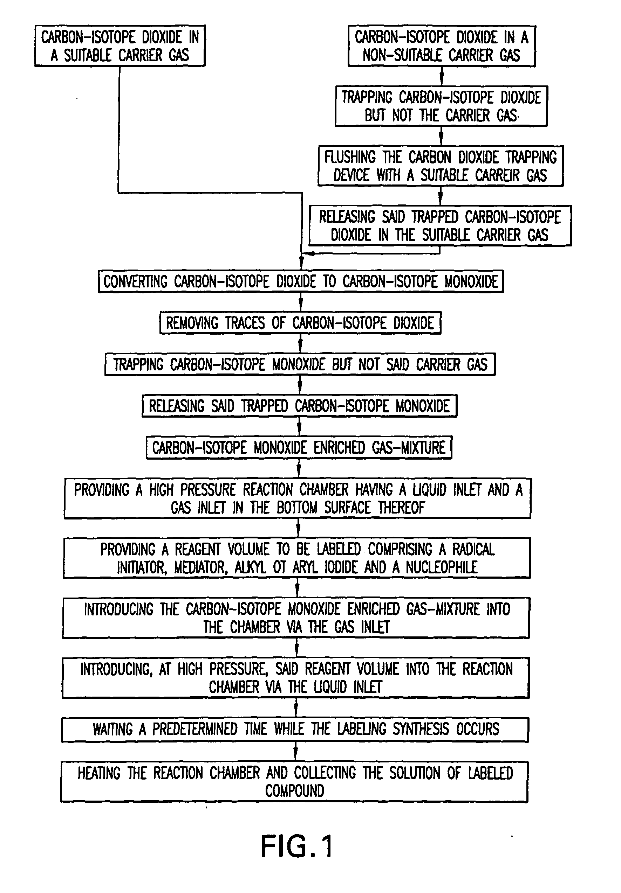

Fig. 1 shows a flow chart over the method according to the invention, which is a labeling, synthesis procedure comprising production of a carbon-isotope monoxide enriched gas mixture. More in detail the production part of the method comprises the steps of:
• Providing carbon-isotope dioxide in a suitable carrier gas of a type that will be described in detail below.
• Converting carbon-isotope dioxide to carbon-isotope monoxide by introducing said gas mixture in a reactor device which will be described in detail below.
• Removing traces of carbon-isotope dioxide by flooding the converted gas-mixture through a carbon dioxide removal device wherein carbon-isotope dioxide is trapped but not carbon-isotope monoxide nor the carrier gas, The carbon dioxide removal device will be described in detail below.
• Trapping carbon-isotope monoxide in a carbon monoxide trapping device, wherein carbon-isotope monoxide is trapped but not said carrier gas. The carbon monoxide trapping device will be described in detail below.
• Releasing said trapped carbon-isotope monoxide from said trapping device, whereby a volume of carbon-isotope monoxide enriched gas-mixture is achieved.

The production step may further comprise a step of changing carrier gas for the initial carbon-isotope dioxide gas mixture if the initial carbon-isotope dioxide gas mixture is comprised of carbon-isotope dioxide and a first carrier gas not suitable as carrier gas for carbon monoxide due to similar molecular properties, such as nitrogen. More in detail the step of providing carbon-isotope dioxide in a suitable second carrier gas such as He, Ar, comprises the steps of:
• Flooding the initial carbon-isotope dioxide gas mixture through a carbon dioxide trapping device, wherein carbon-isotope dioxide is trapped but not said first carrier gas. The carbon dioxide trapping device will be described in detail below.
• Flushing said carbon dioxide trapping device with said suitable second carrier gas to remove the remainders of said first carrier gas.
• Releasing said trapped carbon-isotope dioxide in said suitable second carrier gas.

The labeling synthesis step that may follow the production step utilizes the produced carbon-isotope monoxide enriched gas-mixture as labeling reactant. More in detail the step of labeling synthesis comprises the steps of:
- Providing a high pressure reaction chamber having liquid and gas inlets and in a bottom surface thereof. The reaction chamber will be described in detail below.
- Providing a reagent volume to be labeled comprising a radical initiator, mediator, alkyl or aryl iodide and a nucleophile (e.g., amine, water or alcohol).

A nucleophile is defined as is a reagent that forms a bond to its reaction partner (the electrophile, namely [*carbonyl*-¹¹C]acylating intermediate) by donating both bonding electrons. In preferred embodiments, nucleophiles are amines, water, or alcohols.

An initiator is a substance introduced into a reaction system in order to bring about an initiation reaction. Initiation is a reaction or process generating free radicals which then induce a chain reaction. A thermal radical initiator may be defined as a thermally labile compound that produces free radicals capable of initiating a radical chain reaction.

A mediator is a substance involved in a radical reaction that sustains the chain character of the reaction by making the chain propagation favorable.

Examples of nucleophiles, radical initiator, and mediator will be provided in more detail below.
- Introducing the carbon-isotope monoxide enriched gas-mixture into the reaction chamber via the gas inlet.
- Introducing, at high pressure, said reagent volume into the reaction chamber via the liquid inlet.
- Heating the reaction chamber.
- Waiting a predetermined time while the labeling synthesis occurs.
- Collecting the solution of labeled compound from the reaction chamber.

The solution to be labeled may further comprise bases to facilitate the reaction. Bases are the compounds capable of deprotonating nucleophiles to increase their reactivity. Examples of bases will be provided below.

The step of waiting a predetermined time may further comprise adjusting the temperature of the reaction chamber such that the labeling synthesis is enhanced.

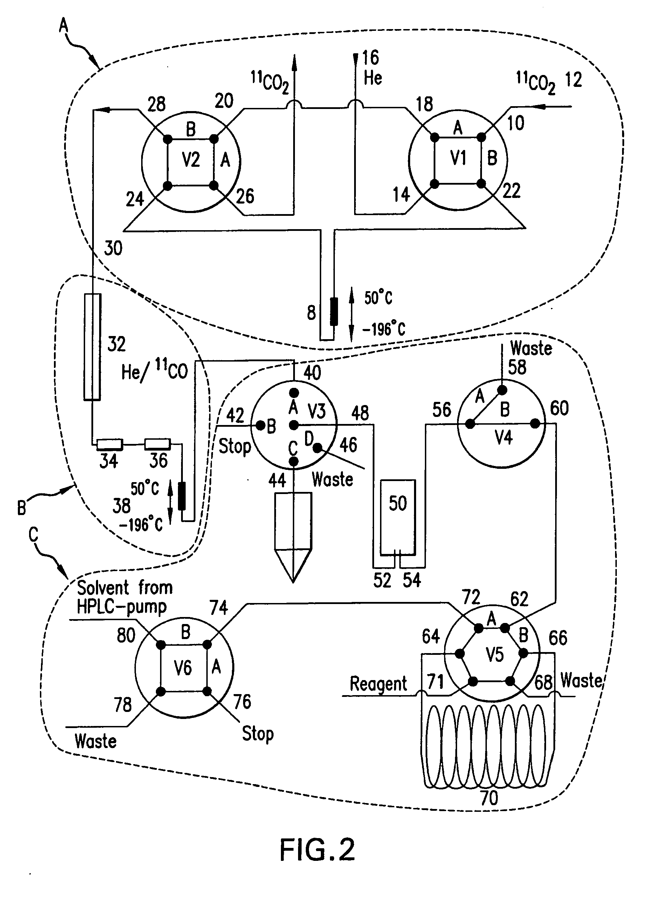

Fig. 2 schematically shows a [¹¹C]carbon dioxide production and labeling-system. The system is comprised of three main blocks, each handling one of the three main steps of the method of production and labeling:
Block A is used to perform a change of carrier gas for an initial carbon-isotope dioxide gas mixture, if the initial carbon-isotope dioxide gas mixture is comprised of carbon-isotope dioxide and a first carrier gas not suitable as carrier gas for carbon monoxide.
Block B is used to perform the conversion from carbon-isotope dioxide to carbon-isotope monoxide, and purify and concentrate the converted carbon-isotope monoxide gas mixture.
Block C is used to perform the carbon-isotope monoxide labeling synthesis.

Block A is normally needed due to the fact that carbon-isotope dioxide usually is produced using the 14N(p,α)¹¹C reaction in a target gas containing nitrogen and 0.1% oxygen, bombarded with 17 MeV protons, whereby the initial carbon-isotope dioxide gas mixture comprises nitrogen as carrier gas. However, compared with carbon monoxide, nitrogen show certain similarities in molecular properties that makes it difficult to separate them from each other, e.g. in a trapping device, whereby it is difficult to increase the concentration of carbon-isotope monoxide in such a gas mixture. Suitable carrier gases may instead be helium or argon.

Block A can also used to change the pressure of the carrier gas (e.g. from 1 to 4 bar), in case the external system does not tolerate the gas pressure needed in block B and C. In an alternative embodiment the initial carbon-isotope dioxide gas mixture is comprised of carbon-isotope dioxide and a first carrier gas that is well suited as carrier gas for carbon monoxide, whereby the block A may be simplified or even excluded.

According to a preferred type of device (Fig. 2), block A is comprised of a first valve V1, a carbon dioxide trapping device 8, and a second valve V2.

The first valve V1 has a carbon dioxide inlet 10 connected to a source of initial carbon-isotope dioxide gas mixture 12, a carrier gas inlet 14 connected to a source of suitable carrier gas 16, such as helium, argon and the like. The first valve V1 further has a first outlet 18 connected to a first inlet 20 of the second valve V2, and a second outlet 22 connected to the carbon dioxide trapping device 8. The valve V1 may be operated in two modes A, B, in mode A the carbon dioxide inlet 10 is connected to the first outlet 18 and the carrier gas inlet 14 is connected to the second outlet 22, and in mode B the carbon dioxide inlet 10 is connected to the second outlet 22 and the carrier gas inlet 14 is connected to the first outlet 18.

In addition to the first inlet 20, the second valve V2 has a second inlet 24 connected to the carbon dioxide trapping device 8. The second valve V2 further has a waste outlet 26, and a product outlet 28 connected to a product inlet 30 of block B. The valve V2 may be operated in two modes A, B, in mode A the first inlet 20 is connected to the waste outlet 26 and the second inlet 24 is connected to the product outlet 28, and in mode B the first inlet 20 is connected to the product outlet 28 and the second inlet 24 is connected to the waste outlet 26.

The carbon dioxide trapping device 8 is a device wherein carbon dioxide is trapped but not said first carrier gas, which trapped carbon dioxide thereafter may be released in a controlled manner. This may preferably be achieved by using a cold trap, such as a column containing a material which in a cold state, (e.g. -196°C as in liquid nitrogen or -186°C as in liquid argon) selectively trap carbon dioxide and in a warm state (e.g. +50°C) releases the trapped carbon dioxide. (In this text the expression "cold trap" is not restricted to the use of cryogenics. Thus, materials that traps the topical compound at room temperature and release it at a higher temperature are included). Examples of suitable material are silica and porapac Q®. The trapping behavior of a silica-column or a porapac-column is related to dipole-dipole interactions or possibly Van der Waal interactions. The said column 8 is preferably formed such that the volume of the trapping material is to be large enough to efficiently trap (>95%) the carbon-isotope dioxide, and small enough not to prolong the transfer of trapped carbon dioxide to block B. In the case of porapac Q® and a flow of 100 ml nitrogen/ min, the volume should be 50-150 µl. The cooling and heating of the carbon dioxide trapping device 8 may further be arranged such that it is performed as an automated process, e.g. by automatically lowering the column into liquid nitrogen and moving it from there into a heating arrangement.

According to the preferred type of device of Fig. 2, block B is comprised of a reactor device 32 in which carbon-isotope dioxide is converted to carbon-isotope monoxide, a carbon dioxide removal device 34, a check-valve 36, and a carbon monoxide trapping device 38, which all are connected in a line.

The reactor device 32 is a reactor furnace comprising a material that when heated to the right temperature interval converts carbon-isotope dioxide to carbon-isotope monoxide, wherein said material is zinc or molybdenum or any other element or compound with similar reductive properties. If the reactor device 32 is a zinc furnace it should be heated to 350 to 400°C, and it is important that the temperature is regulated with high precision. The melting point of zinc is 420°C and the zinc-furnace quickly loses it ability to transform carbon dioxide into carbon monoxide when the temperature reaches over 410 °C, probably due to changed surface properties. The material should be efficient in relation to its amount to ensure that a small amount can be used, which will minimize the time needed to transfer radioactivity from the carbon dioxide trapping device 8 to the subsequent carbon monoxide trapping device 38. The amount of material in the furnace should be large enough to ensure a practical life-time for the furnace (at least several days). In the case of zinc granulates, the volume should be 100-1000 µl.

The carbon dioxide removal device 34 is used to remove traces of carbon-isotope dioxide from the gas mixture exiting the reactor device 32. In the carbon dioxide removal device 34, carbon-isotope dioxide is trapped but not carbon-isotope monoxide nor the carrier gas. The carbon dioxide removal device 34 may be comprised of a column containing ascarite® (i.e. sodium hydroxide on silica). Carbon-isotope dioxide that has not reacted in the reactor device 32 is trapped in this column (it reacts with sodium hydroxide and turns into sodium carbonate), while carbon-isotope monoxide passes through. The radioactivity in the carbon dioxide removal device 34 is monitored as a high value indicates that the reactor device 32 is not functioning properly.

Like the carbon dioxide trapping device 8, the carbon monoxide trapping device 38, has a trapping and a releasing state. In the trapping state carbon-isotope monoxide is selectively trapped but not said carrier gas, and in the releasing state said trapped carbon-isotope monoxide is released in a controlled manner. This may preferably be achieved by using a cold trap, such as a column containing silica or materials of similar properties, such as molecular sieves. Such a cold trap selectively traps carbon monoxide in a cold state below -100°C, e.g. -196°C as in liquid nitrogen or -186°C as in liquid argon, and releases the trapped carbon monoxide in a warm state (e.g. +50°C). The trapping behavior of the silica-column is related to dipole-dipole interactions or possibly Van der Waal interactions. The ability of the silica-column to trap carbon-isotope monoxide is reduced if the helium, carrying the radioactivity, contains nitrogen. A rationale is that since the physical properties of nitrogen are similar to carbon monoxide, nitrogen competes with carbon monoxide for the trapping sites on the silica.

According to the preferred type of device of Fig. 2, block C is comprised of a first and a second reaction chamber valve V3 and V4, the aforementioned reaction chamber 50, a reagent valve V5, an injection loop 70 and a solvent valve V6.

The first reaction chamber valve V3 has a gas mixture inlet 40 connected to the carbon monoxide trapping device 38, a stop position 42, a collection outlet 44, a waste outlet 46, and a reaction chamber connection port 48 connected to a gas inlet 52 of the reaction chamber 50. The first reaction chamber valve V3 has four modes of operation A to D. The reaction chamber connection port 48 is: in mode A connected to the gas mixture inlet 40, in mode B connected to the stop position 42, in mode C connected to the collection outlet 44, and in mode D connected to the waste outlet 46.

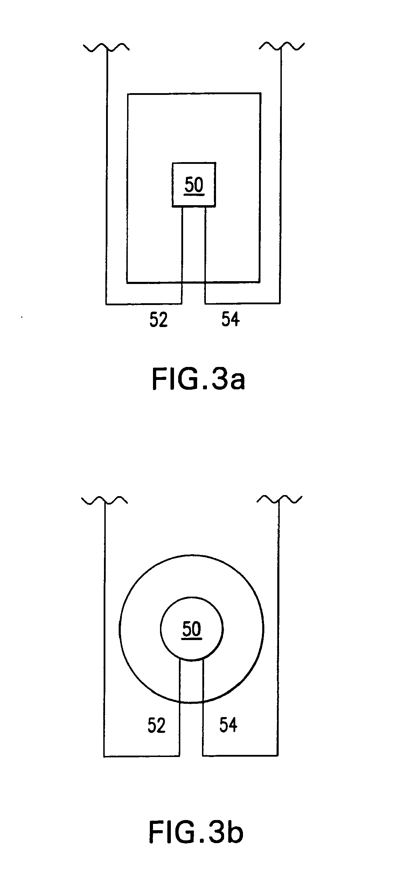

The reaction chamber 50 (micro-autoclave) has a gas inlet 52 and a liquid inlet 54, which are arranged such that they terminate at the bottom surface of the chamber. Gas inlet 52 may also be used as product outlet after the labeling is finished. During operation the carbon-isotope monoxide enriched gas mixture is introduced into the reaction chamber 50 through the gas inlet 52, where after the solution to be labeled with transition metal complex at high pressure enters the reaction chamber 50 through the liquid inlet 54. Fig. 3a and 3b shows schematic views of two preferred reaction chambers 50 in cross section. Fig 3a is a cylindrical chamber which is fairly easy to produce, whereas the spherical chamber of fig. 3b is the most preferred embodiment, as the surface area to volume-ratio of the chamber is further minimized. A minimal surface area to volume-ratio optimizes the recovery of labeled product and minimizes possible reactions with the surface material. Due to the "diving-bell construction" of the reaction chamber 50, both the gas inlet 52 and the liquid inlet 54 becomes liquid-filled and the reaction chamber 50 is filled from the bottom upwards. The gas-volume containing the carbon-isotope monoxide is thus trapped and given efficient contact with the reaction mixture. Since the final pressure of the liquid is approximately 80 times higher than the original gas pressure, the final gas volume will be less than 2 % of the liquid volume according to the general gas-law. Thus, a pseudo one-phase system will result. In the instant application, the term "pseudo one-phase system" means a closed volume with a small surface area to volume-ratio containing >96% liquid and <4% gas at pressures exceeding 200 bar. In most syntheses the transfer of carbon monoxide from the gas-phase to the liquid phase will probably not be the rate limiting step. After the labeling is finished the labeled volume is nearly quantitatively transferred from the reaction chamber by the internal pressure via the gas inlet/product outlet 52 and the first reaction chamber valve V3 in position C.

The second reaction chamber valve V4 has a reaction chamber connection port 56, a waste outlet 58, and a reagent inlet 60. The second reaction chamber valve V4 has two modes of operation A and B. The reaction chamber connection port 56 is: in mode A connected to the waste outlet 58, and in mode B it is connected to the reagent inlet 60.

The reagent valve V5, has a reagent outlet 62 connected to the reagent inlet 60 of the second reaction chamber valve V4, an injection loop inlet 64 and outlet 66 between which the injection loop 70 is connected, a waste outlet 68, a reagent inlet 71 connected to a reagent source, and a solvent inlet 72. The reagent valve V5, has two modes of operation A and B. In mode A the reagent inlet 71 is connected to the injection loop inlet 64, and the injection loop outlet 66 is connected to the waste outlet 68, whereby a reagent may be fed into the injection loop 70. In mode B the solvent inlet 72 is connected to the injection loop inlet 64, and the injection loop outlet 66 is connected to the reagent outlet 62, whereby reagent stored in the injection loop 70 may be forced via the second reaction chamber valve V4 into the reaction chamber 50 if a high pressure is applied on the solvent inlet 72.

The solvent valve V6, has a solvent outlet 74 connected to the solvent inlet 72 of the reagent valve V5, a stop position 76, a waste outlet 78, and a solvent inlet 80 connected to a solvent supplying HPLC-pump (High Performance Liquid Chromatography) or any liquid-pump capable of pumping organic solvents at 0-10 ml/ min at pressures up to 400 bar (not shown). The solvent valve V6, has two modes of operation A and B. In mode A the solvent outlet 74 is connected to the stop position 76, and the solvent inlet 80 is connected to the waste outlet 78. In mode B the solvent outlet 74 is connected to the solvent inlet 80, whereby solvent may be pumped into the system at high pressure by the HPLC-pump.

Except for the small volume of silica in the carbon monoxide trapping device 38, an important difference in comparison to the carbon dioxide trapping device 8, as well as to all related prior art, is the procedure used for releasing the carbon monoxide. After the trapping of carbon monoxide on carbon monoxide trapping device 8, valve V3 is changed from position A to B to stop the flow from the carbon monoxide trapping device 38 and increase the gas-pressure on the carbon monoxide trapping device 38 and increase the gas-pressure on the carbon monoxide trapping device 38 to the set feeding gas pressure (3-5 bar). The carbon monoxide trapping device 38 is then heated to release the carbon monoxide from the silica surface while not significantly expanding the volume of carbon monoxide in the carrier gas. Valve V4 is changed from position A to B and valve V3 is then changed from position B to A. At this instance the carbon monoxide is rapidly and almost quantitatively transferred in a well-defined micro-plug into the reaction chamber 50. Micro-plug is defined as a gas volume less than 10% of the volume of the reaction chamber 50, containing the topical substance (e.g. 1-20 µL). This unique method for efficient mass-transfer to a small reaction chamber 50, having a closed outlet, has the following prerequisites:
• A micro-column 38 defined as follows should be used. The volume of the trapping material (e.g. silica) should be large enough to efficiently trap (>95%) the carbon-isotope monoxide, and small enough (< 1% of the volume of a subsequent reaction chamber 50) to allow maximal concentration of the carbon-isotope monoxide. In the case of silica and a reaction chamber 50 volume of 200 µl, the silica volume should be 0.1-2 µl.
• The dead volumes of the tubing and valve(s) connecting the silica column and the reaction chamber 50 should be minimal (<10% of the micro-autoclave volume).
• The pressure of the carrier gas should be 3-5 times higher than the pressure in the reaction chamber 50 before transfer (101.32501 kPa (1 atm.)).

In one specific preferred type of device, materials and components are chosen as follows. High pressure valves from Valco®, Reodyne® or Cheminert® are used. Stainless steel tubing with o.d. 1/16" is used except for the connections to the porapac-column 8, the silica-column 38 and the reaction chamber 50 where stainless steel tubing with o.d. 1/32" are used in order to facilitate the translation movement. The connections between V1, V2 and V3 should have an inner diameter of 0.2-1 mm. The requirement is that the inner diameter should be large enough not to obstruct the possibility to achieve the optimal flow of He (2-50 ml/ min) through the system, and small enough not to prolong the time needed to transfer the radioactivity from the porapac-column 8 to the silica-column 38. The dead volume of the connection between V3 and the autoclave should be minimized (< 10% of the autoclave volume). The inner diameter (0.05-0.1 mm) of the connection must be large enough to allow optimal He flow (2-50 ml/ min). The dead volume of the connection between V4 and V5 should be less than 10% of the autoclave volume.

When column 8 is a porapac-column, it is preferably comprised of a stainless steel tube (o.d.= 1/8", i.d.= 2 mm, 1= 20 mm) filled with Porapac Q® and fitted with stainless steel screens. The silica-column 38 preferably is comprised of a stainless steel tube (o.d= 1/16", i.d.= 0.1 mm) with a cavity (d=1 mm, h= 1 mm, V= 0.8 µl) in the end. The cavity is filled with silica powder (100/80 mesh) of GC-stationary phase type. The end of the column is fitted against a stainless steel screen.

It should be noted that a broad range of different materials could be used in the trapping devices. If a GC-material is chosen, the criterions should be good retardation and good peak-shape for carbon dioxide and carbon monoxide respectively. The latter will ensure optimal recovery of the radioactivity.

Below a detailed description is given of a method of producing carbon-isotope using an exemplary system as described above.

Preparations of the system are performed by the steps 1 to 5:
1. V1 in position A, V2 in position A, V3 in position A, V4 in position A, helium flow on with a max pressure of 5 bar. With this setting, the helium flow goes through the [¹¹C] carbon dioxide trapping column, the zinc furnace, the [¹¹C] carbon monoxide trapping column, the reaction chamber 50 and out through V4. The system is conditioned, the reaction chamber 50 is rid of solvent and it can be checked that helium can be flowed through the system with at least 10 ml/min.
2. The zinc-furnace is turned on and set at 400°C.
3. The [¹¹C] carbon dioxide and [¹¹C] carbon monoxide trapping columns are cooled with liquid nitrogen. At -196°C, the porapac-and silica-column efficiently traps carbon-isotope dioxide and carbon-isotope monoxide respectively.
4. V5 in position A (load). The injection loop (250 µl), attached to V5, is loaded with the reaction mixture.
5. The HPLC-pump is attached to a flask with freshly distilled THF (or other high quality solvent) and primed. V6 in position A.

Production of carbon-isotope dioxide may be performed by the steps 6 to 7:
6. Carbon-isotope dioxide is produced using the 14N(p,α) ¹¹C reaction in a target gas containing nitrogen (AGA, Nitrogen 6.0) and 0.1 % oxygen (AGA. Oxygen 4.8), bombarded with 17 MeV protons.
7. The carbon-isotope dioxide is transferred to the apparatus using nitrogen with a flow of 100 ml/min.

Synthesis of carbon-isotope may thereafter be performed by the steps 8 to 16
8. V1 in position B and V2 in position B. The nitrogen flow containing the carbon-isotope dioxide is now directed through the porapac-column (cooled to -196 °C) and out through a waste line. The radioactivity trapped in the porapac-column is monitored.
9. When the radioactivity has peaked, V1 is changed to position A. Now a helium flow is directed through the porapac-column and out through the waste line. By this operation the tubings and the porapac-column are rid of nitrogen.
10. V2 in position A and the porapac-column is warmed to 50 °C. The radioactivity is now released from the porapac-column and transferred with a helium flow of 10 ml/ min into the zinc-furnace where it is transformed into carbon-isotope monoxide.
11. Before reaching the silica-column (cooled to -196 °C), the gas flow passes the ascarite-column. The carbon-isotope monoxide is now trapped on the silica-column. The radioactivity in the silica-column is monitored and when the value has peaked, V3 is set to position B and then V4 is set to position B.
12. The silica-column is heated to approximately 50 °C, which releases the carbon-isotope monoxide. V3 is set to position A and the carbon-isotope monoxide is transferred to the reaction chamber 50 within 15 s.
13. V3 is set to position B, V5 is set to position B, the HPLC-pump is turned on (flow 7 ml/ min) and V6 is set to position B. Using the pressurized THF (or other solvent), the reaction mixture is transferred to the reaction chamber 50. When the HPLC-pump has reached its set pressure limit (e.g 40 Mpa), it is automatically turned off and then V6 is set to position A.
14. The reaction chamber 50 is moved into the cavity of a heating block containing a high boiling liquid (e.g. polyethylene glycol or mineral oil). The temperature of the heating block is usually in the range of 100-200°C.
15. After a sufficient reaction-time (usually 5 min), V3 is set to position C and the content of the reaction chamber 50 is transferred to a collection vial.
16. The reaction chamber 50 can be rinsed by the following procedure: V3 is set to position B, the HPLC-pump is turned on, V6 is set to position B and when maximal pressure is reached V6 is set to position A and V3 is set to position 3 thereby transferring the rinse volume to the collection vial.

With the recently developed fully automated version of the reaction chamber 50 system, the value of [¹¹C]carbon monoxide as a precursor for ¹¹C-labelled tracers has become comparable with [¹¹C]methyl iodide. Currently, [¹¹C]methyl iodide is the most frequently used ¹¹C-precursor due to ease in production and handling and since groups suitable for labeling with [¹¹C]methyl iodide (e.g. hetero atom bound methyl groups) are common among biologically active substances. Carbonyl groups, that can be conveniently labeled with [¹¹C]carbon monoxide, are also common among biologically active substances. In many cases, due to metabolic events in vivo, a carbonyl group may even be more advantageous than a methyl group as labeling position. The use of [¹¹C]carbon monoxide for production of PET-tracers may thus become an interesting complement to [¹¹C]methyl iodide. Furthermore, through the use of similar technology, such a method will most likely be applicable for synthesis of ¹³C and ¹⁴C substituted compounds.

The main advantages of the present invention are to overcome the limitations of transition metal-mediated reaction to synthesize ¹¹C-labeled compounds using alkyl/aryl iodides and alcohols as precursors and to allow the use of photosensitive precursors and the generation of photosensitive radiolabeled compounds. The levels of specific radioactivity are high compared with alternative methods such as the use of Grignard reactions for preparation of [carbonyl-¹¹C] compounds.

General reaction scheme for the synthesis of labeled compounds are as illustrated below: 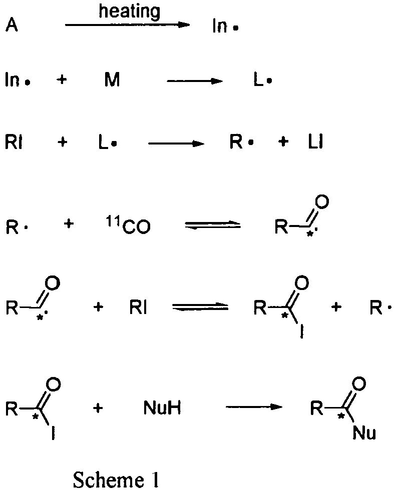 wherein and * indicates the ¹¹C-labeled position; A is a radical thermal initiator; In is a radical(s) produced by A; M is a radical mediator; L. is a radical resulting from the reaction of In and M; RI is an alkyl or aryl iodide and NuH is a nucleophile. Definitions and examples of reaction precursors are as follows.

An initiator (A) is a substance introduced into a reaction system in order to bring about an initiation reaction. Initiation is a reaction or process generating free radicals (In.) which then induce a chain reaction (Scheme 1). A thermal radical initiator (A) may be defined as a thermally labile compound that produces free radicals capable of initiating a radical chain reaction. Preferred examples of radical initiators are:
• Alkyl peroxides: 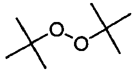 tert-Butyl peroxide
• Acyl peroxides: 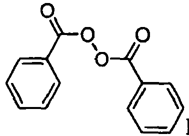 Dibenzoyl peroxide
• Peresters: 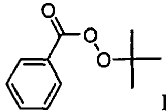 Benzenecarboperoxoic acid tert-butyl ester
• Azo compounds: 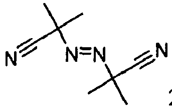
2,2'-Azobis(2-methylpropionitrile)

A mediator (M) is a substance involved in a radical reaction that sustains the chain character of the reaction. In the above Scheme 1, In· is not capable of abstracting iodine atom from RI. Instead In· abstracts atom or group from the mediator M to form reactive radical L· that in turn reacts with the alkyl iodide RI abstracting the iodine atom. In this way the overall conversion becomes favorable. Radical mediators may be compounds with weakly bonded hydrogen atom, which is abstracted by In· (the radical formed on the thermal decomposition of A, step 1), and are exemplified by:
• Organometallic or elementorganic compounds: 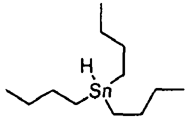 Tributyltin hydride 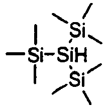 Tris(trimethylsilyl)silane

A nucleophile (NuH) is defined as a reagent that forms a bond to its reaction partner (the electrophile, namely [*carbonyl-*¹¹C]acylating intermediate) by donating both bonding electrons. In the preferred embodiment nucleophiles are amines, water, and alcohols. Nucleophiles (NuH) may be, but not limited to, heteroatom nucleophiles, for example, amines (Nu=R'R"N), water (Nu=OH) or alcohols (Nu=R"'O).

Amines used as precursors in the instant invention have a formula 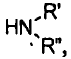 wherein R' and R" are independently H, linear or cyclic lower alkyl or substituted alkyl, aryl or substituted aryl, and may contain, chloro or fluoro groups.

Alcohols used as precursors in the invention have a formula of R"'OH, and may be a primary, secondary or tertiary alcohol, or phenol, wherein R'" may be linear or cyclic alkyl or substituted alkyl, aryl or substituted aryl, and may contain chloro-, fluoro-, keto-, and carboxyl groups.

Iodides used in this invention have a formula RI, where R is linear or cyclic alkyl or substituted alkyl, aryl or substituted aryl, and may contain chloro-, fluoro-, keto-, and carboxyl groups, which are separated by at least one carbon atom from the carbon atom bearing the iodide atom.

The resultant labeled compounds have a formula 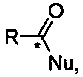 wherein R is defined as above and Nu are defined as a deprotonated nucleophile. Examples of the labeled compounds are: (1) amides of a formula 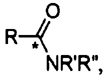 (2) acids of a formula 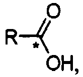 and (3) esters of a formula 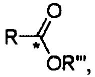 wherein R, R', R" and R"' are defined as above.

In alternative embodiments, additives (e.g. bases) may be used to facilitate the labeling reactions. A base is defined as any organic or inorganic compound that produces Nu anion upon the reaction with amines, water, or alcohols (but preferably does not produce any other products which may be reactive towards reagents, intermediates, and products that will hinder the desired radiolabelling transformation). Examples of base include alkali metal hydrides (for example, KH, NaH), hydroxides (for example, KOH), carbonates (for example, K₂CO₃, Cs₂CO₃), alkali metal amides (for example, lithium hexamethyldisilylamide), alkyl or aryl metals (for example, butyl lithium, phenyl lithium). Nucleophiles are pretreated by such a base before mixing with RI. The term "pretreat" is meant such a base dissolves in or reacts with nucleophiles.

The labeled amides, acids, and esters provide valuable PET tracers in various PET studies.

### Examples

The invention is further described in the following examples.

### Example I - Precursors and Resultant Products

Precursors that were used to label compounds are shown in List. 1.

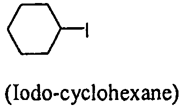

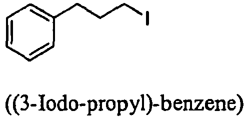

### List 1a. Iodides used as precursors in the synthesis of labeled compounds

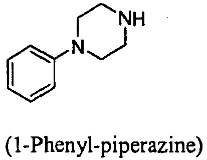

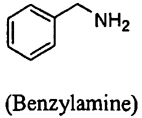

H₂O
(Water)
CH₃OH
(Methanol)

### List 1b. Nucleophiles used as precursors in the synthesis of labeled compounds

The following experiments illustrate the present invention. Radical carbonylation using submicromolar amounts of [¹¹C]carbon monoxide is performed yielding labeled with the compounds shown in Table 1 as target compounds.

**Table 1 Radiochemical yields for ¹¹C-labelled compounds**

| n | Labelled compound^{a} | Solvent | Initiator/mediator | ¹¹CO conv. (%) | Purity (%) | Yield^{b} (%) | Isolated Yield (%) |
|---|---|---|---|---|---|---|---|
| 1 | 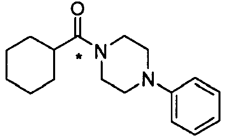 | NMP | 2, 2'-Azobisisobutyron itrile (AIBN) /TTMSS^{c} | 77 | 83 | 64 | - |
| 2 | 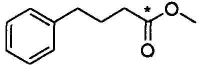 | THF/ metha nol | 1,1'-Azobis(cyclohexa necarbonitrile) / TTMSS | 39 | 66 | 26 | - |
| 3 | 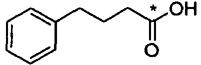 | THF / water | 2,2'-Azobisisobutyron itrile (AIBN) /TTMSS^{c} | 48 | 58 | 28 | - |
| 4 | 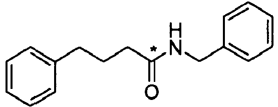 | THF | 1,1'-Azobis(cyclohexa necarbonitrile) / TTMSS | 30 | 63 | 19 | - |

| | | | | | | | |
|---|---|---|---|---|---|---|---|
| ^{a} The position of ¹¹C label is denoted by an asterisk. ^{b} Decay-corrected radiochemical yield determined by LC. ^{c}Tris(trimethylsilyl)silane | | | | | | | |

### Example 2 - Technical Information

[¹¹C]Carbon dioxide production was performed using a Scanditronix MC-17 cyclotron at Uppsala Imanet. The ¹⁴N(p,α)¹¹C reaction was employed in a gas target containing nitrogen (Nitrogen 6.0) and 0.1% oxygen (Oxygen 4.8), that was bombarded with 17 MeV protons.

[¹¹C]Carbon monoxide was obtained by reduction of [¹¹C]carbon dioxide as described previously (Kihlberg, T.; Långström, B. *Method and apparatus for production and use of [¹¹C] carbon monoxide in labeling synthesis.* Swedish Pending Patent Application No. 0102174-0).

Liquid chromatographic analysis (LC) was performed with a gradient pump and a variable wavelength UV-detector in series with a β⁺-flow detector. An automated synthesis apparatus, Synthia (Bjurling, P.; Reineck, R.; Wcompoundberg, G.; Gee, A. D.; Sutcliffe, J.; Långström, B. In Proceedings of the VIth workshop on targetry and target chemistry; TRIUMF: Vancouver, Canada, 1995; pp 282-284) was used for LC purification of the labelled products.

Radioactivity was measured in an ion chamber. In the analysis of the ¹¹C-labeled compounds, isotopically unchanged reference substances were used for comparison in all the LC runs.

NMR spectra were recorded at 400 MHz for ¹H and at 100 MHz for ¹³C, at 25°C. Chemical shifts were referenced to TMS via the solvent signals.

LC-MS analysis was performed with electrospray ionization.

Solvents: THF was distilled under nitrogen from sodium/benzophenone; all other solvents were commercial grade. The solvents were purged with helium.

Alkyl iodides were commercially available or otherwise prepared from commercial alkyl bromides by the Finkelstein reaction.

### Example 3 - Preparation of [carbonyl-¹¹C]compounds

General procedure: A nucleophile, an alcohol, water or amine (150 µmol), radical initiator and radical mediator were placed in a capped vial (1 mL, flushed beforehand with nitrogen to remove air) and dissolved in a solvent (500 µL). An iodide (150 µmol) was added to the solution ca. 7 min before the start of the synthesis. The resulting mixture was pressurized (over 40 MPa) into the autoclave, pre-charged with [¹¹C]carbon monoxide (10⁻⁸ -10⁻⁹ mol) and helium gas mixture. The mixture was heated for 5 from 30°C to 150°C. The crude reaction mixture was then transferred from the autoclave to a capped vial, held under reduced pressure. After measurement of the radioactivity the vial was purged with nitrogen and the radioactivity was measured again. The crude product was diluted with acetonitrile (0.6 mL) and injected on the semi-preparative LC.

## Claims

1. A method for labeling synthesis, comprising:
a) providing a high pressure reaction chamber having a liquid inlet and a gas inlet in a bottom surface thereof,
b) providing a reagent volume to be labeled comprising a thermal radical initiator A, mediator M, alkyl or aryl iodide RI and a nucleophile NuH,
c) introducing a carbon-isotope monoxide enriched gas-mixture into the reaction chamber via the gas inlet,
d) introducing, at high pressure, said reagent volume into the reaction chamber via the liquid inlet,
e) heating the reaction chamber and waiting a predetermined time while the labeling synthesis occurs,
f) collecting a solution of labeled compound of Formula (III) from the reaction chamber: 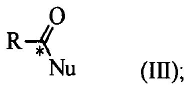
wherein:
R is linear or cyclic alkyl or substituted alkyl, aryl or substituted aryl and may contain chloro-, fluoro-, keto-, and carboxyl groups, which are separated by at least one carbon atom from the carbon atom bearing the iodide atom, and Nu is a deprotonated nucleophile;
wherein high pressure is defined as >30 MPa (>300 bar); and,
wherein the carbon-isotope monoxide is [¹¹C]carbon monoxide.

2. A method of claim 1, wherein the carbon-isotope monoxide enriched gas-mixture is produced by a method comprising:
(a) providing carbon-isotope dioxide in a suitable carrier gas,
(b) converting carbon-isotope dioxide to carbon-isotope monoxide by introducing said gas mixture in a reactor furnace, wherein said reactor furnace comprises a material that when heated to the right temperature interval converts carbon-isotope dioxide into carbon-isotope monoxide, and wherein said material is zinc or molybdenum or any other element or compound with similar reductive properties,
(c) trapping carbon-isotope monoxide in a carbon monoxide trapping device, wherein carbon-isotope monoxide is trapped but not said carrier gas, and
(d) releasing said trapped carbon-isotope monoxide from said trapping device in a well defined micro-plug, whereby a volume of carbon-isotope monoxide enriched gas-mixture is achieved.

3. A method of claim 1, wherein the nucleophile is an amine, water or alcohol.

4. A method of claim 1, wherein the labeled compound is an amide, acid or ester.

5. A method of claim 1, wherein the nucleophile is pretreated with a base.

6. A method of claim 1, wherein the step of introducing the reagent is performed using a pressure that is 80 times higher than the pressure before the introduction, in order to maintain a pseudo one-phase system.

7. A method of claim 1, wherein the step of waiting a predetermined time comprises stirring in the reaction chamber to enhance the labeling synthesis.

8. A method of claim 7, wherein the step of waiting a predetermined time further comprises adjusting the temperature of the reaction chamber so that the labeling synthesis is enhanced.

9. A method of claim 1, wherein the thermal radical initiator A is selected from a list comprising alkyl peroxides, acyl peroxides, perresters and azo compounds.

10. A method of claim 1, wherein the radical mediator is tributyltin hydride or tris(trimethylsilyl)silane.

11. A method of claim 1, wherein the nucleophile is a heteroatom nucleophile.

## Patentansprüche

1. Verfahren zur markierenden Synthese, umfassend:
a) Bereitstellen einer Hochdruckreaktionskammer mit einem Flüssigkeitseinlass und einem Gaseinlass in einer Bodenfläche derselben,
b) Bereitstellen eines zu markierenden Reagensvolumens, das einen thermischen radikalischen Initiator A, einen Vermittler M, Alkyl- oder Aryliodid RI und ein Nukleophil NuH umfasst,
c) Einlassen eines Kohlenstoffisotopmonoxid-angereicherten Gasgemischs in die Reaktionskammer via den Gaseinlass,
d) Einlassen, mit hohem Druck, des Reagensvolumens in die Reaktionskammer via den Flüssigkeitseinlass,
e) Erhitzen der Reaktionskammer und Warten eine vorgegebene Zeit lang, während die markierende Synthese erfolgt,
f) Sammeln einer Lösung von markierter Verbindung der Formel (III) aus der Reaktionskammer: 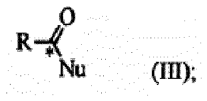
worin:
R für lineares oder cyclisches Alkyl oder substituiertes Alkyl, Aryl oder substituiertes Aryl steht und Chlor-, Fluor-, Keto- und Carboxygruppen enthalten kann, die durch zumindest ein Kohlenstoffatom vom das lodidatom tragenden Kohlenstoffatom getrennt sind, und Nu für ein deprotoniertes Nukleophil steht;
wobei hoher Druck als > 30 MPa (> 300 bar) definiert ist; und
wobei das Kohlenstoffisotopmonoxid [¹¹C]Kohlenstoffmonoxid ist.

2. Verfahren nach Anspruch 1, wobei das Kohlenstoffisotopmonoxid-angereicherte Gasgemisch durch ein Verfahren hergestellt wird, das umfasst:
(a) Bereitstellen von Kohlenstoffisotopdioxid in einem geeigneten Trägergas,
(b) Umwandeln von Kohlenstoffisotopdioxid zu Kohlenstoffisotopmonoxid durch Einlassen des Gasgemischs in einen Reaktorofen, wobei der Reaktorofen ein Material umfasst, das bei Erhitzen auf das richtige Temperaturintervall Kohlenstoffisotopdioxid zu Kohlenstoffisotopmonoxid umwandelt, und wobei das Material Zink oder Molybdän oder irgendein anderes Element oder irgendeine andere Verbindung mit ähnlichen reduktiven Eigenschaften ist,
(c) Fangen von Kohlenstoffisotopmonoxid in einer Kohlenstoffmonoxidfallenvorrichtung, wobei Kohlenstoffisotopmonoxid, aber nicht das Trägergas, gefangen wird, und
(d) Freisetzen des gefangenen Kohlenstoffisotopmonoxids aus der Fallenvorrichtung in einem gut definierten Mikro-Plug, wodurch ein Volumen von Kohlenstoffisotopmonoxid-angereichertem Gasgemisch erzielt wird.

3. Verfahren nach Anspruch 1, wobei das Nukleophil ein Amin, Wasser oder Alkohol ist.

4. Verfahren nach Anspruch 1, wobei die markierte Verbindung ein Amid, eine Säure oder ein Ester ist.

5. Verfahren nach Anspruch 1, wobei das Nukleophil mit einer Base vorbehandelt wird.

6. Verfahren nach Anspruch 1, wobei der Schritt des Einlassens des Reagens durchgeführt wird unter Anwendung eines Drucks, der achtzigmal höher ist als der Druck vor dem Einlassen, um ein Pseudoeinphasensystem aufrechtzuerhalten.

7. Verfahren nach Anspruch 1, wobei der Schritt, bei dem eine vorgegebene Zeit lang gewartet wird, das Rühren in der Reaktionskammer umfasst, um die markierende Synthese zu verbessern.

8. Verfahren nach Anspruch 7, wobei der Schritt, bei dem eine vorgegebene Zeit lang gewartet wird, weiterhin das Regulieren der Temperatur der Reaktionskammer umfasst, damit die markierende Synthese verbessert wird.

9. Verfahren nach Anspruch 1, wobei der thermische radikalische Initiator A aus einer Liste gewählt wird, die Alkylperoxide, Acylperoxide, Perester und Azoverbindungen umfasst.

10. Verfahren nach Anspruch 1, wobei der radikalische Vermittler Tributylzinnhydrid oder Tris(trimethylsilyl)silan ist.

11. Verfahren nach Anspruch 1, wobei das Nukleophil ein Heteroatom-Nukleophil ist.

## Revendications

1. Procédé de synthèse de marquage, comprenant :
a) la préparation d'une chambre de réaction à haute pression comportant une entrée de liquide et une entrée de gaz sur une surface inférieure de celle-ci,
b) la préparation d'un volume de réactif à marquer comprenant un initiateur de radical thermique A, un médiateur M, un iodure d'alkyle ou d'aryle RI un nucléophile NuH,
c) l'introduction d'un mélange gazeux enrichi en monoxyde d'isotope de carbone dans la chambre de réaction par l'entrée de gaz,
d) l'introduction, à haute pression, dudit volume de réactif dans la chambre de réaction par l'entrée de liquide,
e) le chauffage de la chambre de réaction et l'attente pendant une durée prédéterminée que la synthèse de marquage se produise,
f) la collecte d'une solution d'un composé marqué de formule (III) à partir de la chambre de réaction : 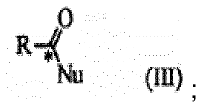
dans laquelle :
R est un alkyle ou alkyle substitué, aryle ou aryle substitué, linéaire ou cyclique et peut contenir des groupes chloro, fluoro, céto et carboxyles, qui sont séparés par au moins un atome de carbone à partir de l'atome de carbone portant l'atome d'iodure, et Nu est un nucléophile déprotoné ;
dans lequel la haute pression est définie comme > 30 MPa (> 300 bars) ; et,
dans lequel le monoxyde d'isotope de carbone est du monoxyde de carbone [¹¹C].

2. Procédé selon la revendication 1, dans lequel le mélange gazeux enrichi en monoxyde d'isotope de carbone est produit par un procédé comprenant :
(a) la préparation de dioxyde d'isotope de carbone dans un gaz porteur approprié,
(b) la transformation du dioxyde d'isotope de carbone en monoxyde d'isotope de carbone en introduisant ledit mélange gazeux dans un four de réaction, dans lequel ledit four de réaction comprend un matériau qui lorsqu'il est chauffée dans la bonne plage de température transforme le dioxyde d'isotope de carbone en monoxyde d'isotope de carbone, et dans lequel ledit matériau est du zinc ou du molybdène ou tout autre élément ou composé avec des propriétés réductrices similaires,
(c) le piégeage du monoxyde d'isotope de carbone dans un dispositif de piégeage de monoxyde de carbone, dans lequel le monoxyde d'isotope de carbone est piégé mais pas le gaz porteur , et
(d) la libération dudit monoxyde d'isotope de carbone piégé à partir dudit dispositif de piégeage dans un micro-tampon bien défini, de telle sorte qu'un volume de mélange gazeux enrichi en monoxyde d'isotope de carbone est obtenu.

3. Procédé selon la revendication 1, dans lequel le nucléophile est une amine, de l'eau ou un alcool.

4. Procédé selon la revendication I, dans lequel le composé marqué est une amide, un acide ou un ester.

5. Procédé selon la revendication 1, dans lequel le nucléophile est préalablement traité avec une base.

6. Procédé selon la revendication 1, dans lequel l'étape d'introduction du réactif est mise en oeuvre en utilisant une pression qui est 80 fois supérieure à la pression avant l'introduction, dans le but de maintenir un système à une pseudo phase unique.

7. Procédé selon revendication 1, dans lequel l'étape d'attente d'une durée prédéterminée comprend l'agitation dans la chambre de réaction afin d'améliorer la synthèse de marquage.

8. Procédé selon la revendication 7, dans lequel l'étape d'attente d'une durée prédéterminée comprend, en outre, l'ajustement de la température de la chambre de réaction de telle sorte que la synthèse marquage est améliorée.

9. Procédé selon la revendication 1, dans lequel l'initiateur de radical thermique A est sélectionné à partir d'une liste comprenant des peroxydes d'alkyle, des peroxydes d'acyle, des peresters et des composés azo.

10. Procédé selon la revendication 1, dans lequel le médiateur de radical est hydrure de tributylétain ou du tris(triméthylsilyle)silane.

11. Procédé selon la revendication 1, dans lequel le nucléophile est un hétéroatome nucléophile.
